# EUROPEAN PATENT SPECIFICATION

(11) **EP 3 424 543 B1**
(45) Date of publication and mention of the grant of the patent: **05.05.2021**
(21) Application number: 16892531.1
(22) Date of filing: 02.03.2016
(51) Int. Cl.: A61B 5/00, A61B 5/20, A61B 5/22, A61M 25/00

(54) **BLADDER URODYNAMIC MEASUREMENT APPARATUS**
URODYNAMISCHE MESSVORRICHTUNG FÜR DIE HARNBLASE
APPAREIL DE MESURE DE LA DYNAMIQUE URINAIRE DE LA VESSIE

(43) Date of publication of application: 09.01.2019
(73) Proprietor: Tsukada Medical Research Co., Ltd., Tokyo 161-0034 (JP)
(72) Inventor: TSUKADA, Osamu, Ueda-shi Nagano 386-2202 (JP); TSUKADA, Manabu, Ueda-shi Nagano 386-2202 (JP); KUSANO, Kazutoshi, Ueda-shi Nagano 386-2202 (JP); SHIMIZU, Masato, Ueda-shi Nagano 386-2202 (JP); NAKASA, Akihiko, Ueda-shi Nagano 386-2202 (JP)
(74) Representative: Barker Brettell LLP
(86) International application number: PCT/JP2016/056327
(87) International publication number: WO 2017/149688

(56) References cited:
- WO-A2-2014/043650
- GB-A- 2 395 128
- JP-A- H10 507 652
- JP-A- 2004 321 296
- JP-A- 2007 523 661
- JP-A- 2008 206 592
- JP-A- 2013 500 802
- JP-A- 2015 500 057
- JP-B2- 2 716 020
- JP-B2- 5 044 115
- JP-B2- 5 249 792
- JP-B2- 5 343 130
- JP-B2- 5 865 513
- US-A1- 2008 027 373
- US-A1- 2012 035 595
- US-A1- 2013 066 166
- US-A1- 2014 200 482
- US-A1- 2015 230 745

## Description

### TECHNICAL FIELD

The present invention relates to a bladder urodynamic measurement apparatus.

### BACKGROUND ART

In general, when urine is stored in a bladder, urine is discharged outside the body by causing the bladder to contract with feeling a desire to urinate as an impetus. However, there are cases where the desire to urinate is unknown and cases where the bladder contracting force is not sufficient to discharge urine even if urine is stored in the bladder, and there are cases where the desire to urinate is strongly felt and contraction of the bladder occurs even if urine is not sufficiently stored in the bladder.

Further, a patient who has lost the contraction function of the bladder itself cannot discharge urine for himself or herself in some cases. In a case like this, a catheter is inserted into the urethra to cause the catheter to reach the inside of the bladder and retain the catheter, and urine in the bladder is discharged through the catheter.

There are patients having a problem of poor contraction of the bladder, and a problem that the desire to urinate and urination pressure do not correspond to each other, and in this case, it is important to measure the pressure in the bladder, the desire to urinate and the urine amount. Similarly, it is important to know the relationship between the desire to urinate and the pressure in the bladder for the patients whose desires to urinate are unknown to discharge urine at a suitable timing. In this regard, there is known an apparatus for temporarily confirming a desire to urinate and the contraction force of the bladder, for example. See Japanese Patent Laid-Open No. 2007-167483.

US 2015/230745 shows a device for detecting bladder pressure having a tube and a pressure gauge. The tube includes an expansion end and a pressure detection end. The expansion end is a closed end and is adapted to be inserted into a bladder to abut against an inner wall of the bladder. The pressure gauge is coupled to the pressure detection end of the tube so as to measure the pressure in the bladder when the expansion end is inserted into the bladder.

US2014/200482 shows systems and methods for monitoring bladder and/or abdominal pressures, and a bladder function recovery device. In the systems and methods, the bladder pressure can be detected in real time with a bladder pressure detection unit that is connected to a urinary catheter. In addition, the abdominal pressure can also be obtained in real time according to the corresponding relationship between the bladder pressure and the abdominal pressure.

### SUMMARY OF INVENTION

### TECHNICAL PROBLEM

However, in order to investigate the relationship among the pressure in the bladder, the desire to urinate and the urine amount of a patient, temporary confirmation is insufficient. By measuring the pressure in the bladder continuously for a short time period to a long time period (24 hours, for example) in the patient's normal life, it becomes possible to evaluate the transition of the pressure in the bladder of the patient, the correlation among the instantaneous pressure rise or the like in the bladder, the desire to urinate and the urine amount. The evaluation result can be used, for example, to evaluate that the patient has an overactive bladder or the like.

The present invention is made in the light of the above-described problem, and an object of the present invention is to provide a bladder urodynamic measurement apparatus that can measure and record pressure in a bladder continuously for a short time period to a long time period.

### SOLUTION TO PROBLEM

One mode of a bladder urodynamic measurement apparatus of the present invention includes a urinary catheter including a catheter main body, a urine discharge part provided at an end portion of the catheter main body, and a branch part branching from the urine discharge part, a pressure sensor provided at the branch part of the urinary catheter and configured to measure a value of pressure that is applied within the urinary catheter by urine in a bladder, a recording device configured to record a pressure value measured by the pressure sensor, and an acceleration sensor, and the recording device is configured to determine a data area of an acceleration at a time of a variation range of the acceleration detected by the acceleration sensor being within a predetermined value, and extract a pressure value that is measured at least partially at a same time as the data area.

In one mode of the bladder urodynamic measurement apparatus of the present invention, the bladder urodynamic measurement apparatus in which the urinary catheter includes a lid for opening and closing the urine discharge part.

In one mode of the bladder urodynamic measurement apparatus of the present invention, the recording device includes a memory for recording the pressure value.

In one mode of the bladder urodynamic measurement apparatus of the present invention, the recording device is configured to record the pressure value cyclically.

In one mode of the bladder urodynamic measurement apparatus of the present invention, the recording device is configured to record a pressure value and a date and time when the pressure value is measured, when the pressure value measured by the pressure sensor exceeds a predetermined value.

In one mode of the bladder urodynamic measurement apparatus of the present invention, the bladder urodynamic measurement apparatus includes a notification section that notifies a user that the pressure value measured by the pressure sensor exceeds a predetermined value, when the pressure value measured by the pressure sensor exceeds the predetermined value.

In one mode of the bladder urodynamic measurement apparatus of the present invention, the recording device is configured to have an external switch, and record a date and time when the external switch is pressed. The invention is defined by appended claims 1-7.

### ADVANTAGEOUS EFFECTS OF INVENTION

According to the present invention, the bladder urodynamic measurement apparatus can be provided, which can measure and record the pressure in a bladder continuously for a short time period to a long time period.

### BRIEF DESCRIPTION OF DRAWINGS

[Fig. 1] Fig. 1 is a schematic view of a bladder urodynamic measurement apparatus according to a present embodiment.
[Fig. 2] Fig. 2 is a schematic block diagram of a recording device illustrated in Fig. 1.

### DESCRIPTION OF EMBODIMENT

Hereinafter, an embodiment of the present invention will be described with reference to the drawings. In the drawings described below, the same or corresponding components will be assigned with the same reference signs and redundant explanation will be omitted.

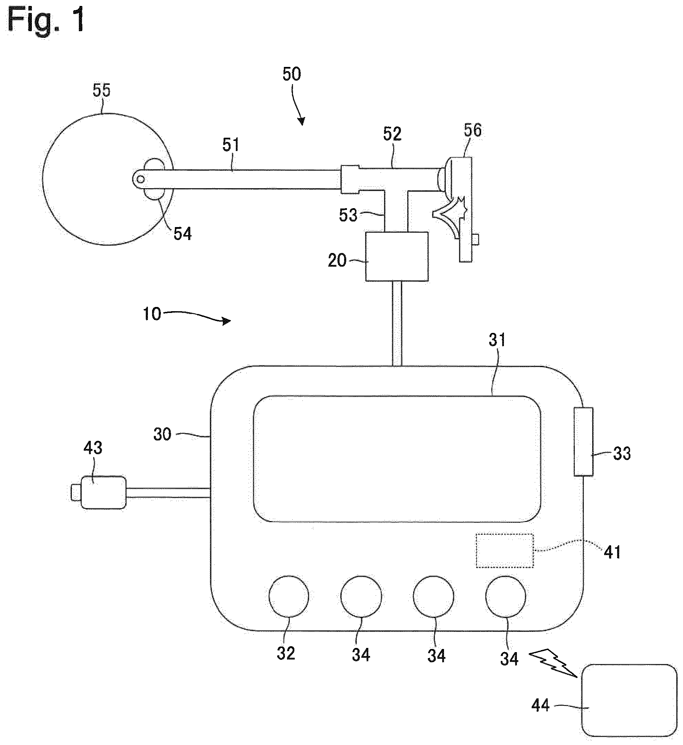

Fig. 1 is a schematic view of a bladder urodynamic measurement apparatus according to the present embodiment. A bladder urodynamic measurement apparatus 10 according to the present embodiment is used in a state where a urinary catheter 50 is inserted in a urethra of a user. Therefore, in Fig. 1, the urinary catheter 50 is also illustrated for convenience of explanation.

First, a configuration of the urinary catheter 50 will be described. The urinary catheter 50 has a catheter main body 51, a tubular urine discharge part 52 that is provided at a rear end portion of the catheter main body 51, and a tubular branch part 53 that branches from a side surface of the urine discharge part 52.

The catheter main body 51 has a balloon 54 at a tip end portion of the catheter main body 51. That is, the urinary catheter 50 is a balloon catheter. The catheter main body 51 is inserted into the urethra of a user in a state where the balloon 54 contracts. After the tip end portion of the catheter main body 51 reaches a bladder 55 of the user, the urinary catheter 50 is retained in the bladder 55 of the user by inflating the balloon 54. Note that in order to inflate the balloon 54, a mechanism that introduces distilled water into the balloon 54 is necessary, but the mechanism is not illustrated in the drawing.

Further, the urinary catheter 50 has a lid 56 for opening and closing a conduit of the urine discharge part 52. The user can discharge urine stored in the bladder 55 from the urine discharge part 52 via the catheter main body 51 by opening the lid 56. The user can stop discharge of urine by closing the urine discharge part 52 with the lid 56 after discharging the urine. When urine starts to be stored in the bladder 55 again, the urine is filled in the catheter main body 51, the urine discharge part 52 and the branch part 53 from a tip end of the catheter main body 51 located in the bladder 55.

Next, the bladder urodynamic measurement apparatus 10 according to the present embodiment will be described. As illustrated in the drawing, the bladder urodynamic measurement apparatus 10 has a pressure sensor 20 configured to measure a value of pressure that is applied to an inside of the urinary catheter 50 by urine in the bladder 55, and a recording device 30 configured to record a pressure value measured by the pressure sensor 20.

The pressure sensor 20 is connected to the branch part 53. The pressure sensor 20 contacts urine filled in the branch part 53 directly or through air, a silicon membrane or silicon oil, and can measure the value of the pressure of the urine which is applied to the inside of the urinary catheter 50. Note that the pressure sensor 20 may be repeatedly used a plurality of times, or may be exchanged for one use.

In one example, the recording device 30 is connected to the pressure sensor 20 by a wire. Note that the recording device 30 may be configured to be communicable with the pressure sensor 20 by radio. The recording device 30 can receive and record the value of the pressure which is applied to the inside of the urinary catheter 50, which is measured by the pressure sensor 20. The recording device 30 has a USB port 33 for transferring the recorded pressure value to an external PC (Personal Computer).

Further, the recording device 30 has a display section 31 for displaying the value, time and the like of the recorded pressure, a switch 32 for starting or stopping recording, and operation sections 34 for performing predetermined input operations to the recording device 30. The recording device 30 is formed to be compact so that the user can easily carry the recording device 30.

In one example, the recording device 30 can have a remote switch 43 that is connected to the recording device 30. The user of the recording device 30 can record a date and time when the remote switch 43 is pressed in the recording device 30, by pressing the remote switch 43 when the user feels a desire to urinate, for example.

Further, in one example, the recording device 30 can have a receiving device 44 for receiving notification from a notification section 40 that will be described later. Thereby, even when a caregiver who cares for the user of the recording device 30 is in a place away from the user, the caregiver can receive notification from the notification section 40 which will be described later.

Further, in one example, the recording device 30 can incorporate an acceleration sensor 41 for measuring a motion state of the user under measurement. The acceleration sensor 41 may be incorporated in the pressure sensor 20 instead of the recording device 30.

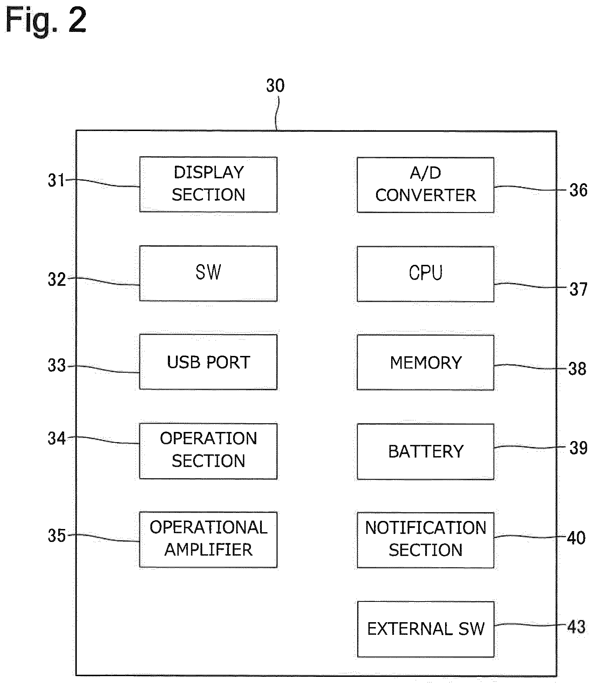

Fig. 2 is a block diagram of the recording device 30. As illustrated in the drawing, the recording device 30 has an operational amplifier 35 for amplifying an analog signal from the pressure sensor 20, an A/D converter 36 that converts an analog signal amplified by the operational amplifier 35 into a digital signal, a CPU 37 for controlling the recording device 30, a memory 38 for recording the pressure value received from the pressure sensor 20, a battery 39 for supplying power to the recording device 30, and the notification section 40 that notifies the user or the caregiver of predetermined information at a time of predetermined conditions, in addition to the display section 31, the switch 32, the USB port 33, the operation sections 34 and the external switch 43 illustrated in Fig. 1.

When the switch 32 is turned on, power is supplied to the recording device 30 and the pressure sensor 20 from the battery 39, and the recording device 30 receives an analog signal indicating the pressure value from the pressure sensor 20. The received analog signal is amplified by the operational amplifier 35. The amplified analog signal is converted into a digital signal by the A/D converter 36. The CPU 37 records the pressure value indicated by the digital signal in the memory 38 cyclically in accordance with a recording cycle set in advance. In one example, the pressure value is recorded in the memory 38 at a cycle of once per second. The recording cycle can be properly changed via the operation section 34. Note that the pressure value may be recorded in an external memory such as a flash memory, instead of the memory 38.

The CPU 37 can cyclically record the measured pressure value in the memory 38, and cause the display section 31 to display the pressure value. In one example, the display section 31 can display the pressure value as a numeric value. In another example, the display section 31 can display the pressure value as a graph.

The recording device 30 can transfer the pressure value recorded in the memory 38 to an external PC connected to the USB port 33. The pressure value which is transferred to the external PC can be properly displayed and analyzed in the external PC.

Further, the recording device 30 can set a value which is inputted by the operation section 34 to the memory 38 as a pressure threshold value. When the pressure value which is measured by the pressure sensor 20 exceeds the set pressure threshold value, the recording device 30 can record the pressure value and the date and time when the pressure value is measured. That is, the recording device 30 can record a pressure value and the date and time when the pressure value is measured in the memory 38 when the pressure sensor 20 measures the pressure value that is a predetermined value or more, in addition to cyclically recording the pressure value.

Further, when the pressure value measured by the pressure sensor 20 exceeds the set pressure threshold value, the recording device 30 can notify the user or the caregiver that the pressure value measured by the pressure sensor 20 exceeds the set pressure threshold value by the notification section 40. In one example, the notification section 40 is a light emitter such as an LED (Light Emitting Diode), a sound generator that generates a beep sound or the like, a communication instrument for transmitting a signal to the receiving device 44 (refer to Fig. 1) or the like, or the like. Accordingly, the notification section 40 can perform the above-described notification to the user or the caregiver by the light emitter flashing, generating a beep sound or the like by the sound generator, or transmitting a signal to the receiving device 44 (refer to Fig. 1) by the communication instrument.

Note that in the present embodiment, the notification section 40 is described as what is provided in the recording device 30, but regardless of this, the pressure sensor 20 may have the notification section 40. Further, in Fig. 1, the notification section 40 is not illustrated.

Next, a method for using the present bladder urodynamic measurement apparatus 10 will be described. The bladder urodynamic measurement apparatus 10 can be used, for example, in a first mode of measuring a urination cycle pressure, a second mode of detecting an abnormal bladder pressure, a third mode of performing urination notification, and a fourth mode of measuring urination pressure.

In the first mode of measuring urination cycle pressure, the user firstly wears the urinary catheter 50 illustrated in Fig. 1, and the pressure sensor 20 of the bladder urodynamic measurement apparatus 10 is connected to the branch part 53. The pressure sensor 20 of the bladder urodynamic measurement apparatus 10 measures a dynamic state of the value of the pressure which is applied to the inside of the urinary catheter 50 by urine in the bladder 55 of the user, in daily life of the user. The recording device 30 records the pressure value measured by the pressure sensor 20 in the memory 38. The recording device 30 may record the pressure value in the memory 38 at long measuring intervals in units of minutes, or may record the pressure value in the memory 38 at short measuring intervals in units of seconds.

Further, the recording device 30 can record the date and time when the remote switch 43 is pressed, in the memory 38, by pressing the remote switch 43 when the user feels a desire to urinate or the like, for example. Accordingly, the recording device 30 can record a pressure value at a time of the remote switch 43 being pressed and a date and time of the pressure value in the memory 38 by associating the pressure value with the date and time. Thereby, a relationship between the date and time when a desire to urinate or the like is felt, and the pressure value which is measured by the pressure sensor 20 can be known.

According to the first mode, the bladder urodynamic measurement apparatus 10 measures the value of the pressure which is applied to the inside of the urinary catheter 50 by urine in the bladder 55 of the user, so that a physiological saline solution for measurement is not required as in the fourth mode which will be described later. Consequently, the bladder urodynamic measurement apparatus 10 can measure, and record the dynamic state of urine pressure following the urination cycle of the user in the daily life of the user continuously and cyclically for a long time period (24 hours, for example). Data of the dynamic state of the urine pressure for a long time period can be used in diagnosis, treatment and the like of a bladder malfunction, an overactive bladder and the like. Note that when the present measurement is performed, a urine collection bag may be attached to the discharge port of the urine discharge part 52.

In the second mode of detecting an abnormal bladder pressure, the user firstly wears the urinary catheter 50 illustrated in Fig. 1, and the pressure sensor 20 of the bladder urodynamic measurement apparatus 10 is connected to the branch part 53. The pressure sensor 20 cyclically measures the dynamic state of the value of pressure that is applied to the inside of the urinary catheter 50 by urine in the bladder 55 of the user as in the first mode. The CPU 37 of the recording device 30 monitors the measured pressure value, and when the CPU 37 determines that the pressure value exceeds a value (an abnormal bladder pressure threshold value) that is set in advance in the memory 38, the CPU 37 records the pressure value and a date and time when the pressure value is measured in the memory 38.

According to the second mode, the date and time when the abnormal pressure occurs to the bladder 55 and the pressure value can be measured, so that a measurement result that can be used to diagnose and treat an overactive bladder can be obtained.

In the third mode of performing urination notification, the user firstly wears the urinary catheter 50 illustrated in Fig. 1, and the pressure sensor 20 of the bladder urodynamic measurement apparatus 10 is connected to the branch part 53. The pressure sensor 20 cyclically measures a dynamic state of a value of pressure that is applied to the inside of the urinary catheter 50 by urine in the bladder 55 of the user as in the first mode and the second mode. The CPU 37 of the recording device 30 monitors a measured pressure value, and when the CPU 37 determines that the pressure value exceeds a value (a urination pressure threshold value) that is set in advance in the memory 38, the CPU 37 notifies the user that the pressure value exceeds the value set in advance in the memory 38 by the notification section 40.

According to the third mode, when the measured pressure value exceeds the urination pressure threshold value, the user is notified that the measured pressure value exceeds the urination pressure threshold value, so that the user can be notified of a suitable urination timing. Accordingly, the user who cannot feel a desire to urinate even when urine is stored in the bladder 55 can be urged to urinate at a suitable timing. Note that as the set value as the urination pressure threshold value, a value lower than the above-described abnormal bladder pressure threshold value is generally set. Further, the urination pressure threshold value can be set in advance based on the dynamic state of the urine pressure measured in the first mode.

When the bladder urodynamic measurement apparatus 10 is used in the third mode, the pressure sensor 20 is preferably configured to be communicable with the recording device 30 by radio. In this case, the pressure sensor 20 is supplied with power from the battery incorporated in the pressure sensor 20, and has an indicator that displays remaining power of the battery. Further, the measured pressure value is transmitted to the recording device 30 by radio from the pressure sensor 20. The notification section 40 performs notification to the user based on the pressure value which the recording device 30 receives from the pressure sensor 20.

In the fourth mode of measuring urination pressure, the user firstly wears the urinary catheter 50 illustrated in Fig. 1, and the pressure sensor 20 of the bladder urodynamic measurement apparatus 10 is connected to the branch part 53. Subsequently, the lid 56 of the urine discharge part 52 is opened, and a prescribed amount of physiological saline solution is injected into the bladder 55 through the urinary catheter 50. After the prescribed amount of physiological saline solution is injected into the bladder 55, the user performs a urination action. At this time, a dynamic state of a value of pressure that is applied to the inside of the urinary catheter 50 by the physiological saline solution in the bladder 55 of the user is measured by the pressure sensor 20 for a period after the physiological saline solution is injected to the bladder 55 until the physiological saline solution is discharged. The pressure value measured by the pressure sensor 20 is cyclically recorded in the memory 38.

According to the fourth mode, urination desire pressure (FD: First Desire) at a time of a desire to urinate being felt first after the physiological saline solution is injected into the bladder 55, maximum urine pressure at the urination time, urine pressure at a urination start time, and urine pressure at a urination end time can be measured and recorded.

As described above, since the bladder urodynamic measurement apparatus 10 according to the present embodiment can measure the value of the pressure which is applied to the inside of the urinary catheter 50 by the urine in the bladder 55, the urination cycle pressure can be measured in the first mode, the abnormal bladder pressure can be detected in the second mode, and urination notification can be performed in the third mode. Note that the bladder urodynamic measurement apparatus 10 can simultaneously execute the first to third modes described above.

When the value of the pressure which is applied to the inside of the urinary catheter 50 by the urine in the bladder 55 of the user is measured, the value of the pressure which is measured includes abdominal muscle pressure of the user. The abdominal muscle pressure varies in accordance with action of the user, and it is estimated that the abdominal muscle pressure increases when action of the user is active. Accordingly, the value of the pressure which is measured by the pressure sensor 20 when the action of the user is active can be larger than actual pressure that is applied to the inside of the urinary catheter 50 by the urine in the bladder 55 of the user. While the abdominal muscle pressure has a large influence on the measurement value, suitable measurement becomes difficult. Therefore, in the bladder urodynamic measurement apparatus 10 according to the present embodiment, bladder inner pressure data (data of the pressure value which is applied to the inside of the urinary catheter 50 by the urine in the bladder 55 of the user) at a time of the influence which the abdominal muscle pressure of the user has on the pressure value being relatively small is analyzed by using the acceleration sensor 41 illustrated in Fig. 1.

First, the acceleration sensor 41 illustrated in Fig. 1 cyclically detects acceleration thereof. The detected acceleration is recorded in the memory 38 illustrated in Fig. 2, for example. The CPU 37 of the recording device 30 analyzes the recorded acceleration data, and determines a data area in which a time-series variation range of the acceleration is relatively small. Specifically, for example, the CPU 37 can determine a data area in which a maximum value and a minimum value of the variation range of the acceleration is within a predetermined range.

Subsequently, the CPU 37 extracts bladder inner pressure data from a time point a predetermined time earlier (for example, 30 minutes before) from a subject mark portion (a urination mark) in the determined data area, from the memory 38. That is, the CPU 37 extracts the bladder inner pressure data which is detected at least partially at a same time as the determined data area from the memory 38. Note that the subject mark portion is a mark indicating a time point at which the user urinated, and is a mark that is inputted at a urination time by the user or the caregiver in the remote switch 43, and/or a mark that is automatically inputted based on pressure data detected by the pressure sensor 20. The subject mark portion is attached to the acceleration data. The CPU 37 sets the extracted bladder inner pressure data as an analysis object, graphs the data and records the data in the memory 38. Based on the graph, it can be evaluated whether the bladder inner pressure is normal, whether involuntary contraction is seen, whether reduction in bladder function is seen and the like.

While the embodiment of the present invention is described thus far, the embodiment of the invention described above is to facilitate understanding of the present invention, but is not to limit the present invention, which is defined by appended claims 1-7.

### REFERENCE SIGNS LIST

- 10: Bladder urodynamic measurement apparatus
- 20: Pressure sensor
- 30: Recording device
- 38: Memory
- 40: Notification section
- 43: External switch
- 50: Urinary catheter
- 51: Catheter main body
- 52: Urine discharge part
- 53: Branch part
- 56: Lid

## Claims

1. A bladder urodynamic measurement apparatus (10), comprising:
a urinary catheter (50) including a catheter main body (51), a urine discharge part (52) provided at an end portion of the catheter main body, and a branch part (53) branching from the urine discharge part (52),
a pressure sensor (20) provided at the branch part (53) of the urinary catheter and configured to measure a value of pressure that is applied within the urinary catheter by urine in a bladder;
a recording device (30) configured to record a pressure value measured by the pressure sensor (20); **characterised in that** the apparatus includes
an acceleration sensor (41),
and the recording device (30) is configured to determine a data area of an acceleration at a time of a variation range of the acceleration detected by the acceleration sensor being within a predetermined value, and extract a pressure value that is measured at least partially at a same time as the data area.

2. The bladder urodynamic measurement apparatus according to claim 1,
wherein the urinary catheter (50) includes a lid (56) for opening and closing the urine discharge part (52).

3. The bladder urodynamic measurement apparatus according to any one of claims 1 to 2,
wherein the recording device (30) includes a memory (38) for recording the pressure value.

4. The bladder urodynamic measurement apparatus according to any one of claims 1 to 3,
wherein the recording device (30) is configured to record the pressure value cyclically.

5. The bladder urodynamic measurement apparatus according to any one of claims 1 to 4,
wherein the recording device (30) is configured to record the pressure value and a date and time when the pressure value is measured, when the pressure value measured by the pressure sensor (20) exceeds a predetermined value.

6. The bladder urodynamic measurement apparatus according to any one of claims 1 to 5, comprising:
a notification section (40) that notifies a user that the pressure value measured by the pressure sensor (20) exceeds a predetermined value, when the pressure value measured by the pressure sensor (20) exceeds the predetermined value.

7. The bladder urodynamic measurement apparatus according to any one of claims 1 to 6,
wherein the recording device (30) is configured to have an external switch (43), and record a date and time when the external switch is pressed.

## Patentansprüche

1. Urodynamische Messvorrichtung (10) für die Harnblase, umfassend:
einen Blasenkatheter (50), der einen Katheterhauptkörper (51), ein Urinablassteil (52), das an einem Endabschnitt des Katheterhauptkörpers bereitgestellt wird, und ein Abzweigungsteil (53), das von dem Urinablassteil (52) abzweigt, einschließt;
einen Drucksensor (20), der an dem Abzweigteil (53) des Blasenkatheters bereitgestellt wird und ausgestaltet ist, um einen Wert des Drucks zu messen, der innerhalb des Blasenkatheters durch Urin in einer Harnblase ausgeübt wird;
eine Aufzeichnungsvorrichtung (30), die ausgestaltet ist, um einen durch den Drucksensor (20) gemessenen Druckwert aufzuzeichnen, **dadurch gekennzeichnet, dass** die Vorrichtung einschließt:
einen Beschleunigungssensor (41),
und wobei die Aufzeichnungsvorrichtung (30) ausgestaltet ist, um zu ermitteln, dass ein Datenbereich einer Beschleunigung zu einer Zeit einer Variationsbreite der Beschleunigung, die durch den Beschleunigungssensor detektiert wird, innerhalb eines vorbestimmten Werts liegt, und um einen Druckwert zu extrahieren, der mindestens teilweise zur gleichen Zeit wie der Datenbereich gemessen wird.

2. Urodynamische Messvorrichtung für die Harnblase nach Anspruch 1, wobei der Blasenkatheter (50) einen Verschluss (56) zum Öffnen und Schließen des Urinablassteils (52) einschließt.

3. Urodynamische Messvorrichtung für die Harnblase nach einem der Ansprüche 1 bis 2, wobei die Aufzeichnungsvorrichtung (30) einen Speicher (38) zum Aufzeichnen des Druckwerts einschließt.

4. Urodynamische Messvorrichtung für die Harnblase nach einem der Ansprüche 1 bis 3, wobei die Aufzeichnungsvorrichtung (30) ausgestaltet ist, um den Druckwert zyklisch aufzuzeichnen.

5. Urodynamische Messvorrichtung für die Harnblase nach einem der Ansprüche 1 bis 4, wobei die Aufzeichnungsvorrichtung (30) ausgestaltet ist, um den Druckwert und ein Datum und eine Uhrzeit aufzuzeichnen, wann der Druckwert gemessen wurde, wenn der durch den Drucksensor (20) gemessene Druckwert einen vorbestimmten Wert überschreitet.

6. Urodynamische Messvorrichtung für die Harnblase nach einem der Ansprüche 1 bis 5, umfassend ein Benachrichtigungssegment (40), das einen Anwender benachrichtigt, dass der durch den Drucksensor (20) gemessene Druckwert einen vorbestimmten Wert überschreitet, wenn der durch den Drucksensor (20) gemessene Druckwert den vorbestimmten Wert überschreitet.

7. Urodynamische Messvorrichtung für die Harnblase nach einem der Ansprüche 1 bis 6, wobei die Aufzeichnungsvorrichtung (30) so ausgestaltet ist, dass sie einen externen Schalter (43) aufweist und ein Datum und eine Uhrzeit aufzeichnet, wenn der externe Schalter gedrückt wird.

## Revendications

1. Appareil de mesure de la dynamique urinaire de la vessie (10), comprenant :
un cathéter urinaire (50) comprenant un corps principal de cathéter (51), une partie d'évacuation de l'urine (52) prévue au niveau d'une partie d'extrémité du corps principal du cathéter, et une partie de dérivation (53) partant de la partie d'évacuation de l'urine (52), un capteur de pression (20) étant prévu au niveau de la partie de dérivation (53) du cathéter urinaire et configuré pour mesurer une valeur de pression qui est appliquée dans le cathéter urinaire par l'urine dans une vessie ;
un dispositif d'enregistrement (30) configuré pour enregistrer une valeur de pression mesurée par le capteur de pression (20) ; **caractérisé en ce que** l'appareil comprend :
un capteur d'accélération (41),
et le dispositif d'enregistrement (30) est configuré pour déterminer une zone de données d'une accélération à un moment où une plage de variation de l'accélération détectée par le capteur d'accélération se trouve en deçà d'une valeur prédéterminée, et pour extraire une valeur de pression qui est mesurée au moins partiellement en même temps que la zone de données.

2. Appareil de mesure de la dynamique urinaire de la vessie selon la revendication 1,
la sonde urinaire (50) comprenant un couvercle (56) pour ouvrir et fermer la partie d'évacuation de l'urine (52) .

3. Appareil de mesure de la dynamique urinaire de la vessie selon l'une quelconque des revendications 1 et 2,
le dispositif d'enregistrement (30) comprenant une mémoire (38) pour enregistrer la valeur de pression.

4. Appareil de mesure de la dynamique urinaire de la vessie selon l'une quelconque des revendications 1 à 3,
le dispositif d'enregistrement (30) étant configuré pour enregistrer la valeur de pression de manière cyclique.

5. Appareil de mesure de la dynamique urinaire de la vessie selon l'une quelconque des revendications 1 à 4,
le dispositif d'enregistrement (30) étant configuré pour enregistrer la valeur de pression et une date et une heure auxquelles la valeur de pression est mesurée, lorsque la valeur de pression mesurée par le capteur de pression (20) dépasse une valeur prédéterminée.

6. Appareil de mesure de la dynamique urinaire de la vessie selon l'une quelconque des revendications 1 à 5, comprenant :
une section de notification (40) qui informe un utilisateur que la valeur de pression mesurée par le capteur de pression (20) dépasse une valeur prédéterminée, lorsque la valeur de pression mesurée par le capteur de pression (20) dépasse la valeur prédéterminée.

7. Appareil de mesure de la dynamique urinaire de la vessie selon l'une quelconque des revendications 1 à 6,
le dispositif d'enregistrement (30) étant configuré pour avoir un interrupteur externe (43), et enregistrer une date et une heure lorsque l'interrupteur externe est pressé.
